# EUROPEAN PATENT SPECIFICATION

(11) **EP 1 779 112 B1**
(45) Date of publication and mention of the grant of the patent: **12.09.2012**
(21) Application number: 05772105.2
(22) Date of filing: 20.07.2005
(51) Int. Cl.: G01N 33/543, G01N 33/53, G01N 37/00

(54) **METHOD TO MEASURE DYNAMIC INTERNAL CALIBRATION TRUE DOSE RESPONSE CURVES**
VERFAHREN ZUR MESSUNG ECHTER DOSIS-WIRKSTOFF-KURVEN MIT DYNAMISCHER INTERNER KALIBRIERUNG
PROCÉDÉ PERMETTANT DE MESURER DES COURBES DE RÉPONSE DE DOSES RÉELLES D'ÉTALONNAGE INTERNE DYNAMIQUE

(30) Priority: 20.07.2004 CA 2475240
(43) Date of publication of application: 02.05.2007
(73) Proprietor: SQI Diagnostics Systems Inc., Toronto, ON M9W 1A4 (CA)
(72) Inventor: LEA, Peter, Toronto, Ontario M5M 3P5 (CA); DE LEO, Domenica, Toronto, Ontario M6S 3M3 (CA); LIU, Jun, Markham, Ontario L6E 1G5 (CA)
(74) Representative: Thornton, Neil
(86) International application number: PCT/CA2005/001147
(87) International publication number: WO 2006/007726

(56) References cited:
- WO-A1-03/025573
- WO-A1-2005/031355
- WO-A2-03/034026
- US-A- 5 200 312
- US-B1- 6 265 173
- STOLL D ET AL: "PROTEIN MICROARRAY TECHNOLOGY" FRONTIERS IN BIOSCIENCE, FRONTIERS IN BIOSCIENCE, ALBERTSON, NY, US, vol. 7, 1 January 2002 (2002-01-01), pages C13-C32, XP008016809 ISSN: 1093-9946
- JOOS T O ET AL: "A MICROARRAY ENZYME-LINKED IMMUNOSORBENT ASSAY FOR AUTOIMMUNE DIAGNOSTICS" ELECTROPHORESIS, WILEY-VCH VERLAG, WEINHEIM, DE, vol. 21, 2000, pages 2641-2650, XP001053250 ISSN: 0173-0835
- TEMPLIN M F ET AL: "PROTEIN MICROARRAYS AND MULTIPLEXED SANDWICH IMMUNOASSAYS: WHAT BEATS THE BEADS?" COMBINATORIAL CHEMISTRY AND HIGH THROUGHPUT SCREENING, HILVERSUM, NL, vol. 7, no. 3, May 2004 (2004-05), pages 223-229, XP008055403 ISSN: 1386-2073
- HUANG R-P: "SIMULTANEOUS DETECTION OF MULTIPLE PROTEINS WITH AN ARRAY-BASED ENZYME-LINKED IMMUNOSORBENT ASSAY (ELISA) AND ENHANCED CHEMILUMINESCENCE (ECL)" CLINICAL CHEMISTRY AND LABORATORY MEDICINE, WALTER DE GRUYTER UND CO, DE, vol. 39, no. 3, March 2001 (2001-03), pages 209-214, XP009022016 ISSN: 1434-6621

## Description

### Field of the Invention

The invention relates to assay devices and methods for constructing assay devices for detecting the presence of an analyte in a biological sample and the quantity of same.

### Background of the Invention

Quality standards for immunoassays have traditionally been driven by external calibration reference standards. Current methods of analysis for typical immunodiagnostic assays provide diagnostic test results based on generally accepted external standard reference measurements. A number of known discrepancies have become apparent to be quantitation errors induced when assays are carried out, leading to variations in test results. For example, the concept of assay sensitivity attempts to characterize sensitivity by classic statistical analysis based on repeated measurement of low concentration samples to confirm that the sample result is not statistically different from zero. As the standard error incurred is inversely proportional to the square root of the number of actual measurements, this method does not actually measure the inherent assay sensitivity. Further refinement has led to some improvements. Known in the art as analytical sensitivity, the zero standard is measured several times and the limit of sensitivity becomes a concentration equating to 2-3 standard deviations (SD) from the mean (M). However, the precision for this theoretical determination may be incorrect by an order of magnitude. The concomitant fitting of any derived external calibration curve(s) does not create a true value dynamic dose response curve that can lead to considerable error in the actual sensitivity.

To further measure the accuracy of such analytical measurement, accuracy is used to define how close the average measured value is to the true value. The difference in measurement is known as the bias or degree of accuracy. Bias may vary over the range of the assay. It is known in the art that methods for measuring this true value need to be developed.

The repeatability of an assay or the estimated error in an analytical assay is known in the art as the percentage coefficient of variation (%CV). Automated assay analysis machines can be affected by variations in sample concentration, temperature, heat and edge effects, incomplete suspension of particles and solid phase precipitation. Precision effects also result from fraction separation and counting errors. In optical systems error is due to effects of turbidity, presence of fluorophores, deterioration of lamps and detectors and the deterioration, over time, of reagents. These factors generally lead to significant decreases in signal to noise ratio. Mechanical manipulation errors can result from poor pipetting and instrument stand-by periods.

As a direct result, the assessment for precision of any analytical method requires the measurement of resulting variability at known and relevant concentrations by using defined or standard control solutions to create baseline calibration standards. Accurate determination of such calibrators is based on measurement of known concentrations in dilution series at predetermined intervals, which are then interpolated. Commercially available, as well as in-house prepared reference solutions or reference standards are available, but are often calibrated with standard or pooled matrices, which may vary considerably from actual patient test samples. Part of the solution to overcome these errors is to plot the precision against a wide range of concentrations to obtain a precision profile, or calibration, of the assay.

Cross reactivity, assay specificity, bias causing interference, alterations in antigen, antibody, binding sites, low dose (competitive assay) and high dose (sandwich assay) hook effects, heterophilic antibody interference, endogenous interfering auto-antibodies, complement, rheumatoid factor, interference in solid phase antibody binding, endogenous signal generating substances, enzyme inhibitors, catalysts and co-factors have also been shown to express confounding activity in assays, including cross reactivity, matrix effects and carry over of sample in automated immunoassay instruments and samplers.

For diagnostic applications, the quality control samples may not reflect actual clinical concentrations in the patient, may not reflect the spectrum of present analytes and interfere with the sample matrix to no longer reflect the content of the patient samples. The quality control samples may measure performance at discrepant intervals of concentration which may not reflect clinical decision points.

WO03/025573 discloses a solid phase assay device for determining analytes in an aqueous sample, and use of the device in immunochromatographic assays. The device comprises an elongate flow matrix allowing lateral transport of fluid therethrough, with the matrix having a sample application zone and downstream thereof a multi-spot detection zone with immobilised capture agents capable of directly or indirectly binding to the analytes.

WO99/36777 discloses a flow matrix device and associated method for the determination of an analyte in a sample using biospecific affinity reactions. The method includes determining a detectable signal of reagent complexed with the analyte and comparing the signal with one or more calibrator values, each of which corresponds to a standard amount of analyte. The flow matrix device includes one or more zones to which the calibrator is bound.

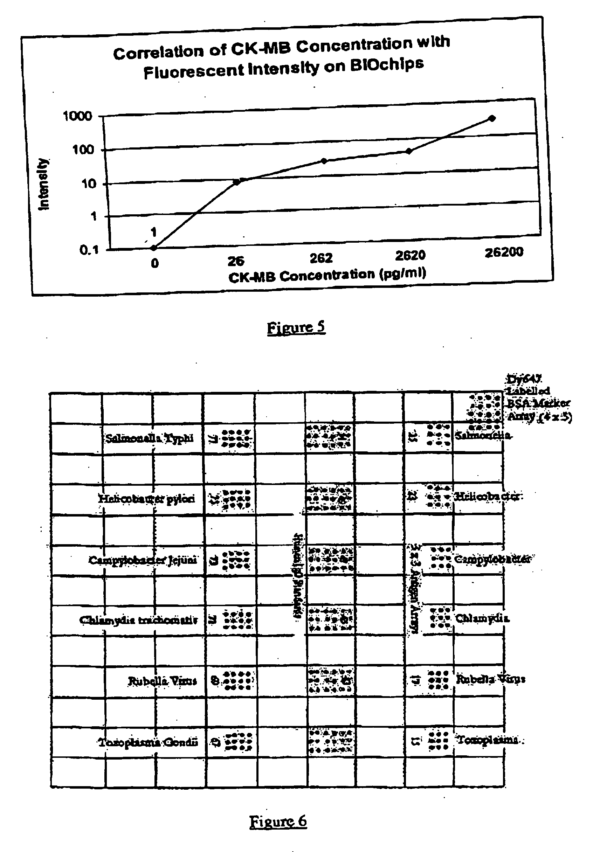

Stoll et al. (2002; Frontiers in Bioscience 7: c13-32) is a review of protein microarray technology. The document discloses in Figure 5 that different fluorescently-labeled proteins can be detected and quantified in parallel using micro-array based assays. Specific capture molecules immobilised in a microarray format bind their respective target protein present in the surrounding solution. Various kinds of control spots, such as positive and negative control spots and/or internal calibration spots, can be included within each microarray.

>

There is therefore a need for an immunoassay that can be reliably calibrated.

### Summary of the Invention

The invention is directed to a method of determining an amount of an analyte in a sample comprising the following steps:
- providing a chip assay device having a surface, said surface having printed thereon a micro-array, said micro-array comprising a plurality of calibration dots and at least one test dot, the calibration dots including differing pre-determined quantities of the analyte, said pre-determined quantities of the analyte providing a concentration of analyte in the calibration dots that corresponds to a dynamic range of the analyte, the test dot including a plurality of capture antibodies for binding to said analyte, said capture antibodies being separated by a non-reactive protein;
- providing a single pre-mixed solution including an antibody that binds specifically to the analyte, said antibody being labeled with a detectable marker;
- introducing the sample into said solution to form a sample solution;
- introducing said sample solution onto said chip assay device;
- measuring an intensity of detectable marker in said calibration dots;
- preparing a calibration curve correlating the amount of analyte in said calibration dots to said intensity of detectable marker;
- measuring an intensity of detectable marker in said at least one test dot; and
- calculating an amount of analyte present in said at least one test dot by comparing the intensity of detectable marker to the amount of analyte corresponding to said intensity in said calibration curve.

Further features of the invention are recited in the appended claims.

The method provides a quantitative analysis that is carried out rapidly using a single assay device with the ability to contain a known minimum volume of test fluid and also to have the ability for flowing fluid through the device in order to meet a known concentration of analyte as a function of analyte concentration per tested volume. Both the calibration dots and test dots are printed within a single assay device which then needs only the application of a single, premixed solution containing the analyte and an excess of detecting reagent which is an antibody.

The invention envisages a method for obtaining dynamic true dose response curves by printing both calibrator and test samples onto a common test platform device. The test platform has a minimum of one test dot. Each test dot has multiple corresponding calibration dots. The signal obtained from the total number of comparative concentration dynamic calibration dots, at indexed X / Y co-ordinates is integrated to form the dynamic internal calibration true dose response curve. In a similar process, the unknown test dot label response reading is also integrated over all obtained readings. The use of multiple test arrays or matrices in platform format predicates a confidence limit approaching one hundred percent in having obtained the correct test result. The common test platform is exposed to the same test fluid and because the calibrator and test samples are exposed simultaneously to the same test fluid, accurate measurement of the concentration of analyte present in the test sample is determined by the resulting true dose response calibration curve. The invention provides the surprising result that the various errors, incurred using known state of the art methods, are not reflected when a test sample is processed with the disclosed method and device.

### Brief Description of the Drawings

Figure 1 is a top view of an assay device of the present invention for carrying out a fixed array test;
Figure 2 is a plot of showing a verification that single and aggregate immuno complexes are quantifiable;
Figure 3 is a plot showing that antigen concentration in the test sample does not impact fluorescence intensity in the calibration spots;
Figure 4 is an illustration of a PicoTip array printing, spot size and array matrices;
Figure 5 is a plot showing a correlation of analyte concentration with fluorescence using dynamic true dose response measurement; and
Figure 6 is top view of an assay device to test for the presence of respective antibody response to micro-organisms having been present in human plasma.

### Detailed Description of the Invention

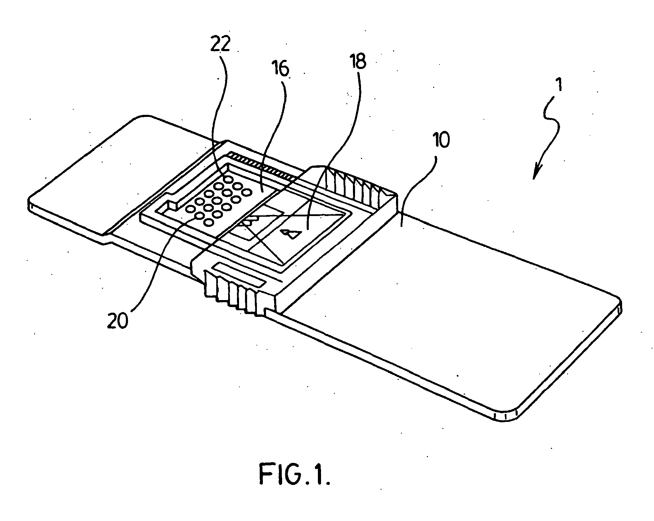

The present method is for calibrating an assay device. A preferred assay device is shown in Figure 1. The assay device has an assay surface 10 that preferably includes a loading area 18 and a reading area 16. The reading area 16 has printed thereon at least one and preferably at least two test dots 20. More preferably, a plurality of dots for detecting the presence of the analyte are printed on the reading area 16. The test dots 20 include a reagent that specifically binds to the protein analyte. Preferably, the reagent is bound antibodies that specifically bind to the analyte. Other reagents known in the art to bind a specific analyte can also be used. For the balance of the present discussion the reagent will be referred to as an antibody.

The bound antibodies are preferably spaced apart to make each bound antibody available for binding to the test antigen free of stearic hindrance from adjacent antigen complexes. A non-reactive protein separates the bound antibodies in the test dots.

The reading area 16 has calibration dots 22 printed thereon. The calibration dots include a pre-determined amount of said analyte for reacting with un-reacted reagent in a vessel, conjugated with a detectable marker. The calibration dots allow the intensity of the label to be correlated to the amount of the antigen present. The intensity of label in the test dots can then be used to derive the quantity of antigen present.

The calibration dots have a concentration of the analyte that corresponds to a dynamic range of the analyte. Dynamic range is the concentration of analyte normally found in the patient. The quantitation needs to be within this lowest and highest concentration and relate to the clinically relevant concentrations.

Many of the problems associated with current methods typical for immunoassays derive from the assay calibration being determined by introducing external standard reference samples for calibration. The present method provides more accurate results by not using these standard external calibration samples.

In developing a platform device for measuring the quantity of a respective analyte, instead of using external standards to generate a calibration or base line, both calibration dots as well as test dots at unknown concentration are printed onto the same test platform. The calibration test dots are printed at known concentrations of analyte. The test dots are printed, also at predetermined X-Y locations, containing only capture reagent which is preferably an antibody specific for the analyte under investigation. The test sample is then conjugated with an excess of marker reagent, which is preferably an antibody, and analyte. The marker antibody has previously been conjugated with a respective fluorescent label, emitting at a suitable wavelength (e.g. 650 nanometers). The marker antibody/antigen complex as well as the free, remaining marker antibody is then flowed over the test platform using laminar flow. A person skilled in the art will appreciate however that other assay devices that do not rely on laminar flow may also be employed. For example, an assay device in the form of a vessel where the antibody/antigen complex as well as the remaining free marker antibody move through the device by diffusion limited kinetics may also be employed for the purposes of the present invention.

In this fashion, marker antibody/unknown concentration of antigen complexes are bound by the capture antibody test dots, whereas free marker antibodies bind to the pre-printed antigen dots at known concentrations. Both the test dots of unknown concentration to be measured and the calibration dots of known concentrations encompassing the dynamic range of the analyte are exposed to the same test fluid sample at the same time. The laminar flow effectively places the analyte components within proximity of the respective binding sites to promote optimal adhesion kinetics for the respective association constants.

The test platform is examined in a reader for determination of the respective concentrations of fluorescent label attached to the dots on the platform when activated by suitable wavelength irradiation. The calibration dots, preferably originally printed at up to ten different concentrations of analyte, result in producing a dynamic internal true dose response curve providing very accurate calibration reference for the assay. The intensity reading obtained from the test dots of unknown concentration is compared to this calibration line. The unknown test concentrations accurately and efficiently interpolate into the dynamic internal true dose response calibration obtained from the known calibration spots.

Each assay device tested provides similarly accurate and reproducible results confirming that the present method for on-platform dynamic calibration provides an accurate, enhanced and sensitive determination of analyte in both quantitative as well as qualitative assays for diagnostic use and detection of analytes in clinical use for humans and animals. This immediate and significant benefit demonstrates that these assays, when processed using the described method (Dynamic Internal Calibration™), do not reflect the errors described in association with current other methods for running these assays while using externally derived calibration standards.

The ability to calibrate and test simultaneously also enhances the confidence level in assuring that the obtained measurements are true. This methodology of the present invention, allows for several different analyte tests to be run on the same assay device at the same time. Several sets of test dots or test arrays for testing for different analytes as well as multiple sets of corresponding calibration dots or calibration arrays for the different analytes can be run on the same assay device at the same time. In order to reach a better than 99% confidence level for a test, the test needs to be run a minimum of three times. Multiple panels of different tests may also be used and run, all at the same time, on a single test platform. The surprising results of the present invention prove that errors in the tests are eliminated, reproducibility is enhanced, the dynamic range for any test is easily extended to cover a required analyte concentration range, sensitivity and coefficient of variance is dramatically improved. The combination of these advantages over prior state of the art also results in considerable saving in time to test results. It is an important advantage that the format of the present invention is device independent and may be applied by those skilled in the art.

The assay device and method as described, effectively represent a novel, quantitative and fast method for the accurate determination of analyte and or marker concentrations, typically for diagnostic clinical markers associated with disease processes. The immediate benefit of rapid, accurate measurement of marker concentration allows for rapid dynamic detection of marker concentration as an indicator of a disease process such as increasing, steady or decreasing concentration, as well as rapid quantitative monitoring of drug efficacy in modifying gene expression for the production of specific marker proteins to indicate drug efficacy.

### Examples

### Example 1: Quantitative Fluorescent Immuno-Assay.

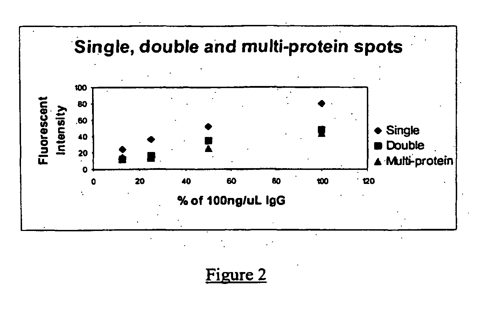

The sandwich immunoassay matrix incorporates a capture antibody that is specific for the antigen of interest and a fluorescence conjugated secondary antibody for detection of analytes and immune complexes. Human chorionic gonadotropin (HCG), a marker of pregnancy in humans, was used as antigen for testing of this platform assay. Currently, the dynamic analytical range for this test is between 1 to 150 fmol/uL (280-37,600mIU/mL) with an assay volume of 5µL. Comparison between the calculated HCG concentration using the device compared with known HCG concentrations has excellent agreement between values (Figure 2, y=1.0717x + 9.9313) and high correlation between mean values for each concentration tested (r=0.9786). Figure 2 is a graph that shows confirmation that single and aggregate immuno complexes provide quantifiable fluorescence when bound to the device platform.

### Example 2: Measurement of antigen dot fluorescence at various sample antigen concentrations.

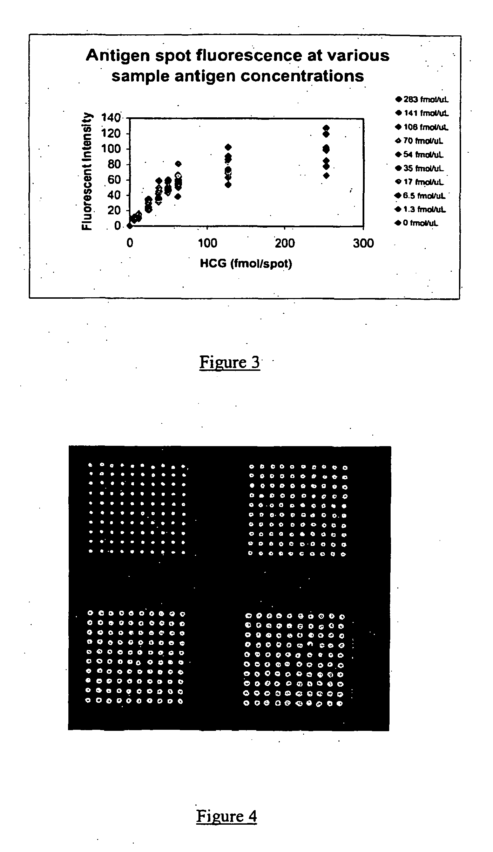

The assay principle is based on quantitative, non-competitive, heterogeneous immunoassays. First generation devices were printed with a series of antigen dots at decreasing concentrations for standard curve auto-calibration; followed by capture antibody dots. Test sample containing the antigen was processed with lyophilized detecting antibody already conjugated to the respective indicator or dye. The test sample reacted with label for 2 minutes and was then dispensed into the chip assay device to be inserted into the reader. The fluorescent intensity of each dot was measured. The reader software compares the fluorescence of the capture antibody dots having unknown antigen concentration with those of the true dose response standard curve having known concentrations and calculates the concentration of the test antigen. Figure 3 is a graph showing data to confirm that antigen concentration in the test sample does not affect the fluorescence intensities of antigen in the calibration dots.

### Example 3: Advanced Array Printing on the Assay Device platform.

Each of four 10 x 10 matrices containing 100 test dots as shown in Figure 4, used 2.6 mm x 2.6 mm of platform area. The center-to-center separation from dot to dot was 260 µm. The number of dispensed droplets per spot increments from 1 to 4 droplet(s) per dot per array matrix. Total chip assay device reading area was 8000 µm x 10000 µm. This printing pattern allowed 1200 dots to be printed on the platform reading area. With a minimum of 5 dots per test, 3 for calibration and 2 test dots, each platform supports 240 tests. This technology advantage allows for fmol/ml antigen detection sensitivity and an increasing number of multiplex test arrays for optimal confidence in diagnostic results by being able to multiplex required test matrices to optimize the calibration curves. Figure 4 provides an illustration of advanced PicoTip Array printing technology on the chip assay device platform.

### Example 4. Correlation of human Para Thyroid Hormone Concentration with Fluorescent Intensity calibrated using True Dose Response Arrays measurement.

As shown in Figure 5, the concentrations of human parathyroid hormone was measured to determine the dynamic internal true dose response calibration curve. The tests confirm that the true dose response was accurately plotted over the dynamic range as tested..

### Example 6: Layout of multiplex format when testing human plasma for 12 different antibodies in response to having been exposed to 12 micro-organisms.

As shown in Figure 6, the test arrays are printed as triplicate arrays at four concentrations for each test, whereas the calibration arrays, in this case for human IgG immunoglobulin, are printed at 6 concentrations in triplicate. An array of 4 dots x 5 dots is used to measure the response of fluorescent label (Dy647) in a binary coding format to confirm the identity of the assay platform when inserted into a reading device.

Those skilled in the art will recognize, or be able to ascertain using no more than routine experimentation, many equivalents to the embodiments of the invention described above. Such equivalents are intended to be encompassed in the scope of the following claims.

## Claims

1. A method of determining an amount of an analyte in a sample comprising the following steps:
(a) providing a chip assay device having a surface, said surface having printed thereon a micro-array, said micro-array comprising a plurality of calibration dots and at least one test dot, the calibration dots including differing pre-determined quantities of the analyte, said pre-determined quantities of the analyte providing a concentration of analyte in the calibration dots that corresponds to a dynamic range of the analyte, the test dot including a first reagent for binding to said analyte;
(b) providing a solution comprising a second reagent that binds specifically to the analyte, said second reagent being labelled with a detectable marker and provided in excess compared to the amount of analyte present in the sample and said calibration dots;
(c) introducing the sample into said solution to form a pre-mixed sample solution;
(d) introducing said pre-mixed sample solution onto said micro-array printed on said chip assay device;
(e) measuring an intensity of the detectable marker in each of said calibration dots;
(f) preparing a calibration curve correlating the amount of analyte in each of said calibration dots to said intensity of the detectable marker in each of said calibration dots;
(g) measuring an intensity of detectable marker in said at least one test dot; and
(h) calculating an amount of analyte present in said at least one test dot by comparing the intensity of the detectable marker in said at least one test dot to the amount of analyte corresponding to said intensity in said calibration curve,
wherein in step (a), said analyte and said first reagent are immobilised to said surface prior to the introduction of said pre-mixed sample solution in step (d).

2. The method according to claim 1 wherein the first reagent is a capture antibody that binds specifically to said analyte and the second reagent is an antibody that binds specifically to said analyte.

3. The method according to claim 1 or claim 2 wherein the assay device has a loading portion for receiving the sample solution and a reading portion, the reading portion having the plurality of calibration dots printed thereon and the test dot printed thereon.

4. The method according to one of claims 1-3 wherein the calibration dots are printed in a volume ranging from I picoliter to I microliter.

5. The method according to claim 4 wherein the volume ranges from 1 picoliter to 4 picoliters.

6. The method according to one of claims 1-5 wherein the calibration dots are printed in a length ranging from 1 picometers to 12 millimeters.

7. The method according to one of claims 1-6 wherein the calibration dots are printed in a length ranging from 25 micrometers to 300 micrometers.

8. The method according to one of claims 1-7 wherein the calibration dots are printed in arrays at predetermined X-Y co-ordinates.

9. The method according to claim 1 wherein the calibration arrays are printed at pre-determined concentrations.

10. The method according to claim 1 wherein the surface of the chip assay device is substantially planar.

11. The method according to claim 1 wherein the calibration dots have a concentration of the analyte that corresponds to a dynamic range of the analyte.

12. The method according to claim 1 wherein the calibration dots are arranged in at least three arrays for carrying out three or more replicates of a test.

13. The method according to claim 1 wherein the test dots are arranged in at least three arrays for carrying out three or more replicates of a test.

14. The method according to claim 12 or claim 13 wherein the test may be the same or different.

15. The method according to claim 1 wherein a single analyte in a single test is measured on the assay device.

16. The method according to claim 1 wherein a single analyte in a plurality of arrays for same test is measured on the assay device.

17. The method according to claim 1 wherein different analytes are measured contemporaneously on the assay device.

18. The method according to claim 1 wherein a plurality of different analytes in a plurality of different tests are measured contemporaneously on the chip assay device.

## Patentansprüche

1. Verfahren zum Bestimmen einer Menge eines Analyten in einer Probe, das die folgenden Schritte beinhaltet:
(a) Bereitstellen einer Chip-Prüfvorrichtung mit einer Oberfläche, wobei die genannte Oberfläche mit einem Mikroarray bedruckt ist, wobei der genannte Mikroarray mehrere Kalibrierungspunkte und wenigstens einen Testpunkt umfasst, wobei die Kalibrierungspunkte verschiedene zuvor festgelegte Mengen des Analyten enthalten, wobei die genannten zuvor festgelegten Mengen des Analyten eine Analytkonzentration in den Kalibrierungspunkten bereitstellen, die einem dynamischen Bereich des Analyten entspricht, wobei der Testpunkt ein erstes Reagenz zur Bindung an den genannten Analyten enthält;
(b) Bereitstellen einer Lösung, die ein zweites Reagenz umfasst, das sich spezifisch an den Analyten bindet, wobei das genannte zweite Reagenz mit einem detektierbaren Marker markiert ist und im Vergleich zu der in der Probe und den genannten Kalibrierungspunkten vorliegenden Analytmenge im Überschuss bereitgestellt wird;
(c) Einleiten der Probe in die genannte Lösung, um eine vorgemischte Probenlösung zu bilden;
(d) Aufbringen der genannten vorgemischten Probenlösung auf den genannten Mikroarray, mit dem die genannte Chip-Prüfvorrichtung bedruckt ist;
(e) Messen einer Intensität des detektierbaren Markers in jedem der genannten Kalibrierungspunkte;
(f) Erstellen einer Kalibrierungskurve, die die Analytmenge in jedem der genannten Kalibrierungspunkte mit der genannten Intensität des detektierbaren Markers in jedem der genannten Kalibrierungspunkte korreliert;
(g) Messen einer Intensität des detektierbaren Markers in dem genannten wenigstens einen Testpunkt; und
(h) Berechnen einer in dem genannten wenigstens einen Testpunkt vorliegenden Analytmenge durch Vergleichen der Intensität des detektierbaren Markers in dem genannten wenigstens einen Testpunkt mit der Analytmenge, die der genannten Intensität in der genannten Kalibrierungskurve entspricht,
wobei in Schritt (a) der genannte Analyt und das genannte erste Reagenz auf der genannten Oberfläche immobilisiert werden, bevor die genannte vorgemischte Probenlösung in Schritt (d) aufgebracht wird.

2. Verfahren nach Anspruch 1, wobei das erste Reagenz ein Fangantikörper ist, der sich spezifisch an den genannten Analyten bindet, und das zweite Reagenz ein Antikörper ist, der sich spezifisch an den genannten Analyten bindet.

3. Verfahren nach Anspruch 1 oder Anspruch 2, wobei die Prüfvorrichtung einen Ladeteil zum Aufnehmen der Probenlösung und einen Leseteil hat, wobei der Leseteil mit den mehreren Kalibrierungspunkten und dem Testpunkt bedruckt ist.

4. Verfahren nach einem der Ansprüche 1-3, wobei die Kalibrierungspunkte in einem Volumen von 1 Pikoliter bis 1 Mikroliter gedruckt sind.

5. Verfahren nach Anspruch 4, wobei das Volumen im Bereich von 1 Pikoliter bis 4 Pikoliter liegt.

6. Verfahren nach einem der Ansprüche 1-5, wobei die Kalibrierungspunkte in einer Länge im Bereich von 1 Pikometer bis 12 Millimeter gedruckt sind.

7. Verfahren nach einem der Ansprüche 1-6, wobei die Kalibrierungspunkte in einer Länge im Bereich von 25 Mikrometern bis 300 Mikrometern gedruckt sind.

8. Verfahren nach einem der Ansprüche 1-7, wobei die Kalibrierungspunkte in Arrays in zuvor festgelegten X-Y-Koordinaten gedruckt sind.

9. Verfahren nach Anspruch 1, wobei die Kalibrierungsarrays in zuvor festgelegten Konzentrationen gedruckt sind.

10. Verfahren nach Anspruch 1, wobei die Oberfläche der Chip-Prüfvorrichtung im Wesentlichen planar ist.

11. Verfahren nach Anspruch 1, wobei die Kalibrierungspunkte eine Analytkonzentration haben, die einem dynamischen Bereich des Analyten entspricht.

12. Verfahren nach Anspruch 1, wobei die Kalibrierungspunkte in wenigstens drei Arrays angeordnet sind, um drei oder mehr Testwiederholungen durchzuführen.

13. Verfahren nach Anspruch 1, wobei die Testpunkte in wenigstens drei Arrays angeordnet sind, um drei oder mehr Testwiederholungen durchzuführen.

14. Verfahren nach Anspruch 12 oder Anspruch 13, wobei der Test gleich oder unterschiedlich sein kann.

15. Verfahren nach Anspruch 1, wobei ein einzelner Analyt in einem einzelnen Test auf der Prüfvorrichtung gemessen wird.

16. Verfahren nach Anspruch 1, wobei ein einzelner Analyt in mehreren Arrays im selben Test auf der Prüfvorrichtung gemessen wird.

17. Verfahren nach Anspruch 1, wobei verschiedene Analyten gleichzeitig auf der Prüfvorrichtung gemessen werden.

18. Verfahren nach Anspruch 1, wobei mehrere verschiedene Analyten in mehreren verschiedenen Tests gleichzeitig auf der Chip-Prüfvorrichtung gemessen werden.

## Revendications

1. Méthode de détermination de la quantité d'un analyte dans un échantillon, comprenant les étapes suivantes :
(a) produire un dispositif de dosage à puce ayant une surface, ladite surface présentant imprimé sur celle-ci un microdamier qui comprend une pluralité de points d'étalonnage et un au moins point de mesure, les points d'étalonnage incluant différentes quantités prédéterminées de l'analyte de façon à obtenir des concentrations en analyte dans les points d'étalonnage qui correspondent à une gamme dynamique de l'analyte et le point de mesure incluant un premier réactif capable de se lier au dit analyte ;
(b) produire une solution comprenant un second réactif qui se lie spécifiquement à l'analyte, ledit second réactif étant marqué au moyen d'un traceur décelable et étant en excès par comparaison à la quantité d'analyte présente dans l'échantillon et dans lesdites points d'étalonnage ;
(c) introduire l'échantillon dans ladite solution pour former une solution échantillon prémélangée ;
(d) introduire ladite solution échantillon prémélangée sur ledit microdamier imprimé sur ledit dispositif de dosage à puce ;
(e) mesurer une intensité du traceur décelable dans chacun desdits points d'étalonnage ;
(f) préparer une courbe d'étalonnage corrélant la quantité d'analyte dans chacun desdits points d'étalonnage à ladite intensité du traceur décelable dans chacun desdits points d'étalonnage ;
(g) mesurer une intensité du traceur décelable dans ledit un au moins point de mesure ; et
(h) calculer la quantité d'analyte présente dans ledit un au moins point de mesure en comparant l'intensité du traceur décelable dans ledit un au moins point de mesure à la quantité d'analyte correspondant à ladite intensité sur ladite courbe d'étalonnage,
dans laquelle à l'étape (a), ledit analyte et ledit premier réactif sont immobilisés sur ladite surface avant l'introduction, à l'étape (d), de ladite solution échantillon prémélangée.

2. Méthode selon la revendication 1, dans laquelle le premier réactif est un anticorps de capture qui se lie spécifiquement au dit analyte et le second réactif est un anticorps qui se lie spécifiquement au dit analyte.

3. Méthode selon la revendication 1 ou la revendication 2, dans laquelle le dispositif de dosage présente une zone de chargement conçue pour recevoir la solution échantillon et une zone de lecture, les points d'étalonnage et le point de mesure étant imprimés sur ladite zone de lecture.

4. Méthode selon l'une des revendications 1-3, dans laquelle les points d'étalonnage sont imprimés à un volume compris entre 1 picolitre et 1 microlitre.

5. Méthode selon la revendication 4, dans laquelle les volumes sont compris entre 1 picolitre et 4 picolitres.

6. Méthode selon l'une des revendications 1-5, dans laquelle les points d'étalonnage sont imprimés à une longueur comprise entre 1 picomètre et 12 millimètres.

7. Méthode selon l'une des revendications 1-6, dans laquelle les points d'étalonnage sont imprimés à une longueur comprise entre 25 micromètres et 300 micromètres.

8. Méthode selon l'une des revendications 1-7, dans laquelle les points d'étalonnage sont imprimés en damiers à des coordonnées X et Y prédéterminées.

9. Méthode selon la revendication 1, dans laquelle les damiers d'étalonnage sont imprimés à des concentrations prédéterminées.

10. Méthode selon la revendication 1, dans laquelle la surface du dispositif de dosage à puce est sensiblement plane.

11. Méthode selon la revendication 1, dans laquelle les points d'étalonnage ont une concentration en l'analyte qui correspond à une gamme dynamique de l'analyte.

12. Méthode selon la revendication 1, dans laquelle les points d'étalonnage sont disposés en au moins trois damiers permettant d'effectuer un test sur au moins trois sous-échantillons.

13. Méthode selon la revendication 1, dans laquelle les points de mesure sont disposés en au moins trois damiers permettant d'effectuer un test sur au moins trois sous-échantillons.

14. Méthode selon la revendication 12 ou la revendication 13, dans laquelle le test peut être le même ou être différent.

15. Méthode selon la revendication 1, dans laquelle un analyte unique est mesuré par un test unique au moyen du dispositif de dosage.

16. Méthode selon la revendication 1, dans laquelle un analyte unique est mesuré dans une pluralité de damiers permettant d'effectuer le même test au moyen du dispositif de dosage.

17. Méthode selon la revendication 1, dans laquelle différents analytes sont mesurés au même moment au moyen du dispositif de dosage.

18. Méthode selon la revendication 1, dans laquelle une pluralité de différents analytes sont mesurés au même moment par une pluralité de tests différents au moyen du dispositif de dosage.
